(19) Europäisches Patentamt
European Patent Office
Office européen des brevets

(11) Veröffentlichungsnummer: **0 343 100 B1**

(12) **EUROPÄISCHE PATENTSCHRIFT**

(45) Veröffentlichungstag der Patentschrift: **06.07.94**

(51) Int. Cl.5: **C12N 9/80**, C12N 1/20, //(C12N1/20,C12R1:06)

(21) Anmeldenummer: **89730123.0**

(22) Anmeldetag: **17.05.89**

# (54) Verfahren zur Isolierung und Charakterisierung eines Gen-Enzymsystems für die Inaktivierung des Herbizids Phenmedipham.

(30) Priorität: **19.05.88 DE 3817384**

(43) Veröffentlichungstag der Anmeldung: **23.11.89 Patentblatt 89/47**

(45) Bekanntmachung des Hinweises auf die Patenterteilung: **06.07.94 Patentblatt 94/27**

(84) Benannte Vertragsstaaten: **AT BE CH DE ES FR GB GR IT LI LU NL SE**

(56) Entgegenhaltungen:

**CHEMICAL ABSTRACTS, Band 107, Nr. 23, 7. December 1987, Seite 252,Zusammenfassung Nr. 213912p, Columbus, Ohio, US; M.K. DERBYSHIRE et al.:"Purification and characterization of an N-methylcarbamate pesticide hydrolyzing enzyme"**

**JOURNAL OF BIOTECHNOLOGY, Band 9, December 1988, Seiten 29-38; ERHARD LEWKE et al.: "Isolation and characterization of enzymes hydrolysing amino acid carbamates"**

**CHEMICAL ABSTRACTS, Band 111, Nr. 11, 11. September 1989, Seite 592, Zusammenfassung Nr. 95488n, Columbus, Ohio, US; E. LEWKE et al.: "Enzymic cleavage of amino acid carbamates"**

(73) Patentinhaber: **SCHERING AKTIENGESELLSCHAFT**

**D-13342 Berlin(DE)**

(72) Erfinder: **Pohlenz, Hans-Dieter, Dr.**
**Danckelmannstrasse 19**
**D-1000 Berlin 19(DE)**
Erfinder: **Boidol, Werner, Dr.**
**Nassauische Strasse 16a**
**D-1000 Berlin 31(DE)**

**EP 0 343 100 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Isolierung und Charakterisierung eines Gen-Enzymsystems für die Inaktivierung des Herbizids Phenmedipham. Das Enzym ist eine Carbamat-Hydrolase aus Arthrobacter oxidans, welche für die Spaltung der -OOC-N- Bindung zwischen den beiden Phenylkernen von Phenmedipham (IUPAC - Bezeichnung für 3-m-Tolylcarbamoyloxyphenylcarbamat) verantwortlich ist.

In der Praxis müssen häufig mehrere Herbizide bzw. Herbizidmischungen zur Bekämpfung verschiedener Unkräuter verwendet werden. Dieses Problem könnte durch gentechnisch veränderte Pflanzen, die eine Resistenz gegen nicht-selektive Herbizide aufweisen, umgangen werden.

Die Forderung nach herbizidtoleranten Pflanzen steht somit heutzutage immer mehr im Vordergrund des Pflanzenschutzes.

Um herbizidtolerante Pflanzen zu erzeugen, bedarf es primär eines Verfahrens zur Isolierung und anschließender Reinigung eines Enzyms, das in der Lage ist, ein Herbizid durch Metabolisierung zu inaktivieren und sekundär eines Verfahrens zur Charakterisierung des Gen-Enzymsystems, das die für für die Codierung des aktiven Enzyms verantwortlichen DNA-Sequenz enthält.

Ein derartiges Verfahren zur Isolierung und anschließenden Charakterisierung eines Gen-Enzymsystems, welches Phenmedipham inaktivieren kann, ist bisher nicht bekannt.

Aufgabe der vorliegenden Erfindung ist es, ein Verfahren zur Isolierung und anschließenden Charakterisierung eines Gen-Enzymsystems, welches Phenmedipham in-aktivieren kann, zu entwickeln. Hierzu wird zunächst ein Screening von Mikroorganismen durchgeführt, um die Mikroorganismen zu identifizieren, die die Fähigkeit besitzen, das Herbizid Phenmedipham durch Metabolisierung zu inaktivieren.

Es wurde nun gefunden, daß sich aus Bodenmikroorganismen, wie Arthrobacter oxidans, eine Carbamat-Hydrolase isolieren läßt, die für die Hydrolyse der -OOC-N- Bindung zwischen den beiden Phenylkernen von Phenmedipham verantwortlich ist, Die Hydrolyse der -OOC-N- Bindung zwischen den beiden Phenylkernen von Phenmedipham führt zu herbizid-inaktiven Verbindungen wie Methyl-3-hydroxyphenylcarbamat und m-Toluidin gemäß folgender Reaktion:

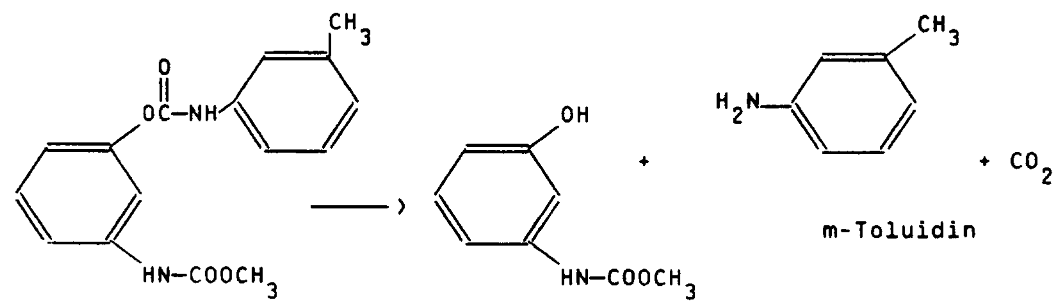

m-Toluidin

Phenmedipham

Methyl-3-hydroxy-phenyl-carbamat

Zur Isolierung und anschließenden Reinigung eines Gen-Enzymsystems, welches Phenmedipham gemäß der oben beschriebenen Reaktion hydrolisieren kann, werden Mikroorganismen von Arthrobacter oxidans in Nährmedien kultiviert. Die für die Spaltung von Phenmedipham verantwortliche Carbamat-Hydrolase wird durch Ultraschall-Zellaufschluß und Zentrifugation isoliert und über Anionenaustausch-Chromatographie, Gradientenelution, Ammoniumsulfatfällung und FPLC-Trennung bis zur elektrophoretischen Homogenität aufgereinigt. Ausgehend von der gereinigten Carbamat-Hydrolase werden nach BrCN-Spaltung zwei Peptide isoliert, deren Sequenz durch Edman-Abbau ermittelt werden kann. Nach Maßgabe der Sequenzinformation dieser Peptide werden Oligonukleotide synthetisiert, die als Hybridisierungssonden zum Nachweis des Carbamat-Hydrolase-Gens verwendet werden.

In allen aufgeführten Isolaten des Bodenbakteriums Arthrobacter oxidans können nach Lyse der Zelle und Extraktion der Nukleinsäuren Plasmide nachgewiesen werden.

Für den Stamm Arthrobacter oxidans P52 kann gezeigt werden, daß die Carbamat-Hydrolase durch ein Plasmid codiert wird.

Bei Verlust des Plasmids pHP52 verliert dieser Stamm die Eigenschaft Phenmedipham hydrolytisch zu spalten. Eine Carbamat-Hydrolase kann in dem plasmidfreien Derivat des Stamms P52 biochemisch nicht nachgewiesen werden.

Das Plasmid pHP52 wird präparativ isoliert und mit Restriktionsendonukleasen kartiert. Die elektrophoretisch aufgetrennten Restriktionsfragmente werden auf Membranfilter transferiert und mit den Oligonukleotiden hybridisiert. Aus den Daten der Blot-Hybridisierungen ergibt sich, daß das Carbamat-Hydrolase-Gen auf einem PstI-Restriktionsfragment der Größe 3,3 kb lokalisiert ist.

Dieses Fragment wird präparativ aus dem Plasmid pHP52 isoliert und in die PstI-Stelle des Vektors pUC19C (Yanish-Perron, C., Vieira, J. & Messing (1985) , Gene 33, 103 ff.) eingebaut. Nach Transformation von E. coli DH5$\alpha$ mit dem Ligierungsansatz werden zwei Typen rekombinanter E. coli Klone erhalten (pp52Pst und pp52Pst inv.), die das Carbamat-Hydrolase-Gen in unterschiedlicher Orientierung zum lac-Promotor des Vektors enthalten (siehe Abbildung 5).

Die Carbamat-Hydrolase wird in Gegenwart des Induktors Isopropyl-$\beta$-D-Thiogalaktosid von Kulturen der Klone des Typs E. coli DH5$\alpha$ (pp52Pst) funktionell exprimiert. In Proteinextrakten von Klonen des Typs E. coli DH5$\alpha$ (pp52 inv.), die das Carbamat-Hydrolase-Gen in inverser Orientierung zum lac-Promotor enthalten, läßt sich keine Expression des Carbamat-Hydrolase-Gens nachweisen. Daraus kann abgeleitet werden, daß der Arthrobacter-Promotor nicht von der E. coli RNA-Polymerase erkannt wird.

Die Nukleotidsequenz des Carbamat-Hydrolase-Gens wird nach der Methode von Sanger et al. (Sanger, F., Nicklen, S, & Coulson, A. (1977), Proc. Natl. Acad, Sci., USA 74, 5463-5468) ermittelt.

Dazu werden ausgehend von dem klonierten 3,3 kb langen PstI-Restriktionsfragment 15 Subklone in den einzelsträngigen DNS-Bakteriophagen M13 mp18 und mp19 (Messing, J. (1983) Methods in Enzymol. 101, 20-78) konstruiert. In Abbildung 6 ist eine genaue Restriktionskarte des codierenden Bereichs dargestellt, aus der die Sequenzierungsstrategie ersichtlich ist.

Die ermittelte Nukleotidsequenz mit der daraus ableitbaren Proteinsequenz ist in Abbildung 7 dargestellt. Die Aminosäuresequenzen der beiden ermittelten BrCN-Spaltpeptide (siehe Beispiel 4) können im selben Leseraster auf DNA-Ebene identifiziert werden, Dieser Leseraster endet mit einem TGA-Translationsstopcodon (siehe Abbildung 7, Nukleotid-Positionen 1789-1791) und beginnt sehr wahrscheinlich mit einem GTG-Startcodon (Abbildung 7, Nukleotid-Positionen 340-342). Insgesamt ergibt sich ein Leseraster von 1479 Basenpaaren. Stromaufwärts des putativen GTG-Startcodons kann eine Region mit signifikanter Homologie zu der Konsensus-Sequenz für E. coli Ribosomenbindungsstellen ("Shine-Dalgarno Box") festgestellt werden (siehe Abbildung 7, Nukleotid-Positionen 298-302).

Bei der Deutschen Sammlung von Mikroorganismen (DSM) in Göttingen, Bundesrepublik Deutschland, wurden folgende Mikroorganismen hinterlegt (Hinterlegungsnummern):

| | | | |
|---|---|---|---|
| Arthrobacter oxidans | P 16/4/B | (DSM 4038) | am 24.03.1987 |
| Arthrobacter oxidans | P 67 | (DSM 4039) | |
| Arthrobacter oxidans | P 75 | (DSM 4040) | |
| Arthrobacter oxidans | P 11/1/-b | (DSM 4041) | |
| Arthrobacter oxidans | P 15/4/A | (DSM 4045) | am 27.03.1987 |
| Arthrobacter oxidans | P 21/2 | (DSM 4046) | |
| Arthrobacter oxidans | P 52, enthaltend das Plasmid pHP52 | (DSM 4044) | |

## Beschreibung der Abbildungen

### Abkürzungen

| | |
|---|---|
| g | - Zentrifugalkraft |
| DEAE | - Diethylaminoethyl |
| FPLC | - (Fast Protein/Peptid/Polynukleotid Liquid Chromatography) = Schnelle Flüssigchromatographie für Proteine, Peptide und Polynukleotide |
| pH | - Negativer dekadischer Logarithmus der Wasserstoffionenkonzentration |
| kb | - Kilobase |
| SDS | - Natriumlaurylsulfat |
| DTT | - Dithiothreit |

1 x SSC - 0.15 M NaCl, 0.015 M Tri-Natriumcitrat pH 7,0
1 x Denhardt - 0,02 % (w/v) Rinderserumalbumin
(Sigma, Fraktion V)
0,02 % (w/v) Ficoll 400
0,02 % Polyvinylpyrrolidon
MCS - Multiple cloning site

**Abkürzungen für Restriktionsendonukleasen**

Bm = BamHI, Bs = BstEII, Cl = ClaI, EV = EcoRV, HII = HindII, Kp = KpnI, Nc = NcoI, Nd = NdeI, Nh = NheI, Ps = PstI, PvI = PvuI, PvII = PvuII, Sc = SacI, Sp = SphI, St = StuI, xb = XbaI.

**Abbildungen**

Abb. 1 - zeigt das Verfahren zur Isolierung und anschließender Reinigung der Phenmedipham spaltenden Carbamat-Hydrolase aus Arthrobacter oxidans (Beispiel 2)
Abb. 2 - zeigt die elektrophoretische Auftrennung des Rohextrakts und der isolierten und gepoolten Proteinfraktionen nach den einzelnen Reinigungsschritten auf einem SDS-Polyacrylamidgel, wobei als Marker für das Molekulargewicht Standardproteine (S) mitgelaufen sind
A : Rohextrakt aus dem Zentrifugationsüberstand des Ultra schall-Zellaufschlusses von Arthrobacter oxidans
B : Gepoolte Proteinfraktion nach Gradientenelution auf einer DEAE-Sephacel-Säule
C : Ammoniumsulfat-Fällung der Fraktion aus B
D : Proteinfraktion nach Gelfiltration der AmmoniumsulfatFällung und anschließender Trennung auf einer Sephacryl S-300 Säule
E : Proteinfraktion nach Trennung der gepoolten Fraktion aus D über FPLC (Anionenaustausch-Chromatographie)
F : Proteinfraktion nach Trennung der gepoolten Fraktion E auf einer Superose 6-Säule (Beispiel 2)
Abb. 3 - zeigt ein Diagramm, aus dem das pH-Optimum (pH 6,8) der Carbamat-Hydrolase entnommen werden kann. Das pH-Optimum läßt sich ermitteln, indem man die Enzymaktivität in % gegen den pH-Wert aufträgt (Beispiel 2)
Abb. 4 - zeigt die Restriktionskarte des Plasmids pHP52 aus Arthrobacter oxidans (Stamm P52).
Abb. 5 - zeigt ein Schema der Klonierung des Carbamat-Hydrolase-Gens. Der Übersicht halber ist der mit dem Oligonukleotiden hybridisierende Genbereich einschließlich der 5'- und 3'-flankierenden Regionen vergrößert unterhalb der ringförmigen Plasmidkarte dargestellt.
Die rekombinanten Klone pp52Pst und pp52Pst inv. sind linear dargestellt. Die Transkriptionsrichtung des lac Z'-Gens ist durch einen Pfeil (➡) angedeutet.
Abb. 6 - zeigt die Restriktionskarte des klonierten 3,3 kb PstI-Restriktionsfragments aus der die genaue Lage des Carbamat-Hydrolase-Gens hervorgeht (Start: GTG = Startcodon, Stop: TGA = Stopcodon).
Die sequenzierten Bereiche sind durch Pfeile unterhalb der Restriktionskarte gekennzeichnet, Aus der Länge der Pfeile kann die Länge der jeweils sequenzierten Bereiche abgelesen werden.
Die Restriktionsfragmente, die die Homologiebereiche zu den in Beispiel 4 beschriebenen Oligonukleotiden enthalten, sind in der Karte hervorgehoben

(–▬–).

Die M13 Klone können wie folgt beschrieben werden:

| Klon | Eingebautes Fragment | M13 Vektor |
|---|---|---|
| A | NdeI/SacI (pUC19)~500 | mp 18 |
| B | NdeI/SacI 1289 bp | mp 18 |
| C | PvuI/PstI ~ 700 bp | mp 19 |
| D | PvuI/PvuI 564 bp | mp 18 |
| E | BamHI/BamHI (pUC 19) ~ 1060 bp | mp 18 |
| F | BamHI/BamHI 958 bp | mp 18 |
| G | PvuI/PvuI 564 bp | mp 18 |
| H | PvuII/SacI 476 bp | mp 18 |
| I | ClaI/BamHI 561 bp | mp 18 |
| K | ClaI/BamHI 397 bp | mp 18 |
| L | PvuII/SacI 476 bp | mp 19 |
| M | KpnI/HindII 445 bp | mp 19 |
| N | BamHI/BamHI 958 bp | mp 18 |
| O | BamHI/BamHI 1100 bp | mp 18 |
| P | KpnI/HindII 445 bp | mp 18 |

Abb. 7 - zeigt die Nukleotidsequenz des Carbamat-Hydrolase-Gens einschließlich der 5'- und 3'-Flankenbereiche.
Besonders gekennzeichnet sind
1. Das GTG-Startcodon
2. Die ribosomale Bindungsstelle
(Shine-/Dalgarno Box; S/D)
3, Die Homologiebereiche zu den in Beispiel 4 beschriebenen Oligonukleotiden
(Oligo I & II/Oligo III).

Die codierende Nukleotidsequenz wurde formal in eine Aminosäuresequenz übersetzt. Der Leseraster ist eindeutig durch die Proteinebene ermittelten Aminosäurepartialsequenzen determiniert.

**Beispiel 1**

Isolierung von Mikroorganismen, die die Fähigkeit besitzen, das Herbizid Phenmedipham zu inaktivieren

Zur Identifzierung von Mikroorganismen, die die Fähigkeit besitzen das Herbizid Phenmedipham durch Metabolisierung zu inaktivieren, wird ein Screening von Mikroorganismen durchgeführt. Als Quelle für die Mikroorganismen werden Bodenproben von unterschiedlichen Standorten (Versuchsfelder, die mehrfach mit Phenmedipham behandelt wurden) und auch Klärschlämme verwendet. Als Selektionskriterien für die Identifizierung von Mikroorganismen, die eine Carbamatabspaltung durchführen können, werden folgende Eigenschaften herangezogen:

a) Wachstum auf Nährmedium mit Phenmedipham als einziger Kohlenstoff- bzw. Stickstoff-Quelle
b) Abbau von Phenmedipham zu gut wasserlöslichen Verbindungen gemäß der folgenden Reaktion:

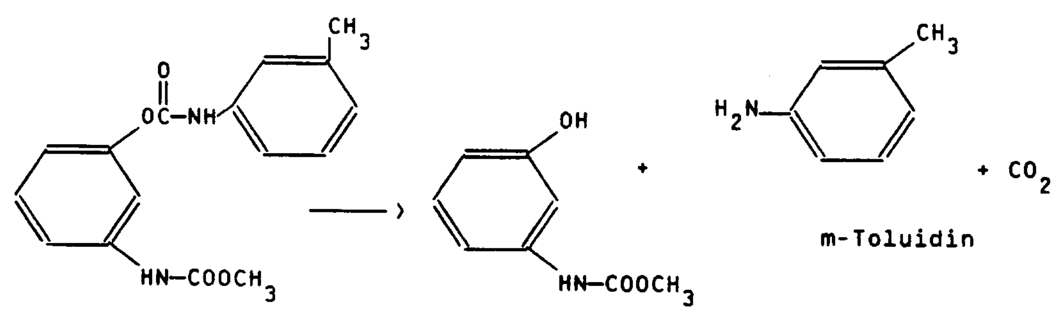

Phenmedipham

Methyl-3-hydroxy-phenyl-carbamat

m-Toluidin

Aus der Vielzahl der in den Bodenproben vorhandenen Mikroorganismen, die zur Spaltung von Phenmedipham befähigt sind, werden sieben Vertreter ausgewählt, die besonders deutlich einen Abbau zeigen. Diese Bodenbakterien, die alle Vertreter der Gattung Arthrobacter und innerhalb dieser Gattung der Spezies oxidans angehören, werden in Kulturbrühen, enthaltend ein synthetisches Medium (M9-Medium) mit

1,0 g/l $NH_4Cl$
0,25 g/l $MgSO_4$ x $7H_2O$
3,0 g/l $KH_2PO_4$
7,0 g/l $Na_2HPO_4$ x $2H_2O$
2,0 g/l Glucose
und 0,5 g/l NaCl,

kultiviert.

Das M9-Medium enthält zusätzlich 1 mg/l Thiamin (Vitamin B1) sowie Spurenelemente, die in Form einer Stammlösung (1 ml/l M9-Medium) zugegeben werden. Diese Spurenelemente-Stammlösung enthält

0,5 g/l Borsäure
0,04 g/l $CuSO_4$ x $5H_2O$
0,2 g/l $FeCl_3$ x $6H_2O$
0,4 g/l $MnSO_4$ x $7H_2O$
0,4 g/l $ZnCl_2$
0,2 g/l $(NH_4)_6Mo_7O_{24}$ x $4H_2O$.

Für Schüttelkulturen im Flüssigmedium wird das synthetische Medium durch 0,1 % Casaminoacids (Difco$^R$) ergänzt.

Die Bodenbakterien werden in diesem M9-Medium bei 28 °C, unter guter Belüftung, bis zum Ende der logarithmischen Wachstumsphase inkubiert. Für eine Enzymaufreinigung werden insgesamt 10 l Medium mit einer stationären Vorkultur 1:100 beimpft.

Durch HPLC-Analyse der Kulturbrühen konnte gezeigt werden, daß in den Kulturen von Arthrobacter oxidans die gewünschte Spaltung von Phenmedipham zu den herbizid inaktiven Produkten vollzogen wird.

## Beispiel 2

Isolierung und Reinigung der Carbamat-Hydrolase aus Arthrobacter oxidans

Die Isolierung und anschließende Reinigung der Carbamat-Hydrolase bis zur elektrophoretischen Homogenität wird über ein 6-stufiges Reinigungsverfahren durchgeführt. Aus 6 Liter einer endlogarithmischen Kultur von Arthrobacter oxidans (pHP52) (DSM Nr. 4044) können 0,5 - 1 mg Carbamat-Hydrolase reproduzierbar isoliert werden. Zur Isolierung der Carbamat-Hydrolase werden die Zellen durch Zentrifugation (7000 x g) geerntet und in ca. 40 ml Aufschlußpuffer (10 mM Natriumphosphat pH 6.8/1 mM DTT) resuspendiert. Die Zellsuspension wird durch Ultraschall aufgeschlossen und gleichzeitig homogenisiert. Das so gewonnene Homogenat wird anschließend 45 Minuten bei 40000 x g und einer Temperatur von 4 °C zentrifugiert. Der Niederschlag wird verworfen, der Überstand auf eine DEAE Sephacel-Säule, äquilibriert mit 100 mM Tris-HCl pH 7,2/100 mM NaCl/1 mM DTT, aufgetragen (Säulendurchmeser 2,6 mm, Höhe des Gelbetts 20,5 cm, Säulenvolumen ca. 100 ml).

Vor dem Auftragen wird der Zellextrakt mit Startpuffer [100 mM Tris/100 mM NaCl/1 mM DTT] ca. 1:10 verdünnt. Die Säule wird anschließend mit Startpuffer gewaschen, um nicht bindendes Material zu entfernen. Die Carbamat-Hydrolase wird dann mit einem linearen Gradienten 100 mM NaCl -> 500 mM NaCl (5 x Säulenvolumen) eluiert. Die enzymatisch aktiven Fraktionen werden gepoolt (vereinigt) und mit festem Ammoniumsulfat [$(NH_4)_2SO_4$] auf eine Endkonzentration von 33 % der gesättigten Lösung versetzt. Der dabei entstehende Proteinniederschlag wird durch Zentrifugation sedimentiert (20000 x g/30 min) und verworfen. Der Überstand wird mit $(NH_4)_2SO_4$ [fest] auf eine Endkonzentration von 60 % der gesättigten Lösung versetzt und ca. 12 h bei 0 °C gerührt. Die ausgefallenen Proteine werden durch Zentrifugation gesammelt (20000 x g/30 min). Der Niederschlag wird in ca. 1 ml Startpuffer gelöst, mit 10 % (w/v) Saccharose versetzt und auf eine Sephacryl S-300 Säule aufgetragen. Die Gelfiltration wird mit einer Flußrate von 2,5 cm/h durchgeführt (Elutionspuffer -> Startpuffer), Die Säule hat die Geometrie: Durchmesser = 2,6 cm h = 95 cm, Volumen = 475 ml. Die enzymatisch aktiven Fraktionen werden anschließend über eine FPLC-Säule
(Mono Q HR 5/5; Anionenaustauscher) weiter aufgearbeitet.
(Gradientenelution: 100 mM NaCl -> 300 mM NaCl; Flußrate: 0,5 ml/mm; Auftragsvolumen: 2 ml).
Die nicht bindenden Proteine werden durch isokratische Elution mit 19 ml Startpuffer entfernt.
(Gradientenelution: 100 mM NaCl -> 300 mM NaCl innerhalb von 20 ml in 100 mM Tris/HCl pH 7,2/1 mM DTT).

Die enzymatisch aktivsten Fraktionen werden nach elektrophoretischer Analyse der Reinheit (SDS-Polyacrylamid-Gelelektrophorese nach Lämmli) durch Ultrafiltration konzentriert (Amicon(R), Centrikon 10 Konzentrator) und auf eine FPLC-Gelfiltrationssäule (Superose 6, HR 10/30, Pharmacia) aufgetragen. (Flußrate: 0,2 ml/min; Auftragsvolumen: 100 $\mu$l; Laufmittel: 100 mM Tris/HCl pH 7,2 / 10 mM NaCl).
Die aktive Proteinfraktion, die bei diesem Schritt anfällt, ist elektrophoretisch einheitlich.

Das isolierte Enzym ist in gepufferten Lösungen (mit in der Biochemie ublichen Puffern, wie Phosphatpuffer, Trispuffer etc.; pH 6,8) aktiv. Kofaktoren oder Metallionen sind für die Reaktion nicht erforderlich, eine Empfindlichkeit gegen SH-Reagenzien liegt ebenfalls nicht vor.
Das pH-Optimum des Enzyms liegt bei pH 6,8.
Sowohl unter denaturierenden/dissoziierenden Bedingungen (SDS-Gelelektrophorese) als auch unter nativen Bedingungen (Gelfiltration) liegt das Molekulargewicht der Carbamat-Hydrolase im Bereich von 50-60 kd, vorzugsweise 53-57 kd. Daraus ist zu folgern, daß es sich bei der Carbamat-Hydrolase um ein monomeres Protein handelt. Der isoelektrische Punkt der CarbamatHydrolase liegt bei pI = 6.2.

## Beispiel 3

Verfahren zum Nachweis der Carbamat-Hydrolase

Zum schnellen und zweifelsfreien Nachweis der Enzymaktivität während der Aufreinigung des Proteinrohextrakts wird ein in vitro-Enzymtest entwickelt. Der Test basiert auf der Eigenschaft des Enzyms, in Wasser schwer lösliches Phenmedipham in lösliche Hydrolyseprodukte umzuwandeln. Dazu wird festes Phenmedipham in Wasser suspendiert und mit Ultraschall mikronisiert. Diese Mikrosuspension wird anschließend bei 50 °C unter Rühren in eine Agaroselösung eingegossen und vor dem Erstarren in Petrischalen gegeben. Es bildet sich eine trübe Gelmatrix. Die Enzymlösung wird anschließend in Stanzlöcher gegeben, die in die feste Matrix eingeführt worden sind. Nach Inkubation der Testplatten für 2 - 4 Stunden bei 30 °C wird die Enzymaktivität durch das Auftreten klarer Zonen in der durch Phenmedipham milchigtrüben Matrix angezeigt.

## Beispiel 4

Identifizierung der Aminosäuresequenz zweier BrCN-Spaltpeptide und Synthese von Oligonukleotiden zum spezifischen Nachweis des Carbamat-Hydrolase-Gens durch Hybridisierung

Ausgehend von der gereinigten Carbamat-Hydrolase werden nach BrCN-Spaltung zwei Peptide isoliert, deren Sequenz durch Edman-Abbau partiell ermittelt werden kann.

BrCN Peptid I:

$H_2N$ - Ser - Asp - Glu - Phe - Ala - Asn - Leu - Asp -
- Arg - Trp - Thr - Gly - Lys - Pro - Phe - Val - Asp (Val) -
- Gly (His) - Leu - Asp - Glu - Val - Ala - Val - COOH

BrCN Peptid II:

$N_2H$ - Glu - His - Thr - Lys - Phe(Val) - Asn(Gly) - Glu - Arg(Cys) -
- Pro - Leu - Ala - Phe - Tyr - Pro -Val - Phe - Asn - Glu - COOH

Nach Maßgabe der Sequenzinformation dieser Peptide werden Oligonukleotide synthetisiert, die als Hybridisierungssonden zum Nachweis des Carbamat-Hydrolase-Gens verwendet werden:
Oligonukleotid I (17 mer, "mixed probe")

enthält als einzelsträngiges DNS-Fragment die Sequenzinformation aus BrCN-Peptid I Aminosäuren Pos. 10-15 (Komplementärstrang).

```
         | A   | C   | A   | A   |
5'- AA   | GGG | TTT | GCC | GGT | CCA-3'

         | C   |     | C   | C   |
         | T   |     | T   | T   |
    Phe  | Pro | Lys | Gly | Thr | Trp
```

Oligonukleotid II (42 mer)
enthält als einzelsträngiges DNS-Fragment die Sequenzinformation aus BrCN-Peptid I Aminosäuren Pos. 8-21 (Komplementärstrang). Die Codonauswahl erfolgt unter Annahme einer Guanin(G)-und Cytosin(C)-reichen DNS-Sequenz (es werden an der dritten Position der Tripletts Guanin(G)- und Cytosin(C)-Nukleotide vor Adenin(A)- und Thymin(T)-Nukleotiden berücksichtigt).

```
5'- CTG | GTC | CAG | GCC | GTC | CAC | GAA |
    Gln | Asp | Leu | Gly | Asp | Val | Phe |

    CGG | CTT | GCC | GGT | CCA | GCG |
    Pro | Lys | Gly | Thr | Trp | Arg |

    GTC | - 3'
    Asp |
```

Oligonukleotid III
enthält als einzelsträngiges DNS-Fragment Sequenzinformation aus BrCN-Peptid II (Komplementärstrang)

```
5'- TTC | GTT | GAA | GAC | CGG | GTA | GAA | CGC |
    Glu | Asn | Phe | Val | Pro | Tyr | Phe | Ala |
```

Unter Verwendung dieser Oligonukleotide ist es möglich, das Herbizidase-Gen innerhalb des Plasmids pHP52 durch Hybridisierung zu lokalisieren. Dazu wird die Plasmid-DNS mit Restriktionsendonukleasen gespalten, die dabei entstehenden Fragmente werden durch Agarosegelelektrophorese aufgetrennt und anschließend nach der Methode von E.M. Southern (J.Mol.Biol. 98, 503-517 (1975)) in einzelsträngiger Form auf Membranfilter transferiert (Gene Screen Plus™ Hybridisierungsmembranen, Du Pont de Nemours/NEN-Research Products).

Die Obligonukleotide werden unter Verwendung von T4-Polynukleotidkinase (Boehringer Mannheim) und [$\gamma$-$^{32}$P]-Adenosin-5'-triphosphat (>5000 Ci/mmol, Du Pont de Nemours/NEN-Research-Products) endmarkiert (R.B. Wallace and C.G. Miyada, Methods in Enzymology, 152, 432-442(1987)) und ohne weitere Aufreinigung für die Hybridisierung eingesetzt.

Die Hybridisierung erfolgt unter Anwendung von Standardverfahren (P.J. Mason & J.G. Williams in "Nucleic acid hybridisation" S. 113-160 (1985) B.D. Hames & S.J. Higgins Hrsg. IRL Press Oxford, Washington D.C.). Unter den Bedingungen 6 x SSC; 10 x Denhardt; 0,5 % (w/v) SDS und 100 $\mu$g/ml t-RNA (Bäckerhefe, Boehringer Mannheim) sowie 10 ng/ml markiertem Oligonukleotid I/II/III bei 41 °C ($\geqq$ 6h) läßt sich eine spezifische Hybridisierung erreichen. Der Nachweis der Hybride erfolgt durch Autoradiographie (T. Maniatis, E.F. Fritsch & J. Sambrook, "Molecular cloning", Cold Spring Harbor Laboratory (1982)).

**Beispiel 5**

Isolierung und Charakterisierung des Plasmids pHP52 aus Arthrobacter oxidans P52

Zur Isolierung des Plasmids pH52 aus Arthrobacter wird die alkalische Extraktionsmethode nach Bimboim und Doly (Birnboim, H.C. & Doly, J. (1979) Nucl. Acid. Res., 7, 1513-1523) mit einer Modifikation von Brandsch und Decker (Brandsch, R. & Decker, K. (1984) Arch. Microbiol. 138, 15-17) angewendet. Für eine Präparation werden die Bakterien in 6 Litern LB-Medium bestehend aus

| | |
|---|---|
| Bacto-Trypton (Difco$^R$) | 10 g/l |
| Bacto-Hefe-Extrakt (Difco$^R$) | 5 g/l |
| NaCl | 10 g/l |

bis zu einer Zelldichte von $OD_{550}$ = 1,4 kultiviert und anschließend durch Zentrifugation geerntet.

Die Zellen werden dann in insgesamt 210 ml Lösung I (50 mM Glukose; 10 mM EDTA; 25 mM Tris/HCl pH 8,0; 1 mg/ml Lysozym) resuspendiert und eine Stunde bei Raumtemperatur inkubiert. Die Lyse erfolgt durch Zugabe von 360 ml Lösung II (0,2 M NaOH; 1 % SDS). Nach vorsichtigem Durchmischen und anschließender Inkubation für 5 Minuten bei Raumtemperatur und anschließendem Abkühlen auf Eis für 5 Minuten wird das Gemisch durch Zugabe von 180 ml Lösung III (2 M Tris/HCl pH 7,0 / 0,5 M KCl) neutralisiert. Nach einer einstündigen Inkubation auf Eis wird das unlösliche Präzipitat durch Zentrifugation entfernt. Die Plasmid-DNS wird durch Zugabe von 0,6 Volumen % Isopropanol zum klaren Überstand gefällt und nach einer Inkubation von 15 Minuten bei Raumtemperatur durch Zentrifugation (15 000 x g/30 Minuten) pelletiert. Der plasmidhaltige Niederschlag wird im Vakuum getrocknet und anschließend in 24 ml 10 x TE Puffer (100 mM Tris/HCl pH 8,0; 10 mM EDTA) gelöst. Diese plasmidhaltige Lösung wird anschließend durch isopyknische Casiumchlorid-Dichtegradientenzentrifugation in Gegenwart von Ethidiumbromid gereinigt (Maniatis, T., Fritsch, E.F, & Sambrook, J. in "Molecular Cloning" (1982), Cold Spring Harbor, N.Y.).

Die gereingite Plasmid-DNS wird anschließend mit Restriktionsendonukleasen durch Einzel- und Mehrfachspaltungen sowie anschließender gelelektrophoretischer Auftrennung der Restriktionsfragmente in 0,8 % (w/v)-Agarosegelen analysiert. Zur Größenbestimmung der Fragmente wird mit den Nukleasen Hind III sowie Hind III/Eco RI behandelte DNS des Bakteriophagen λ als Standard verwendet.

Mit Hilfe dieser Informationen wird eine ringförmige Restriktionskarte des Plasmids pHP52 erstellt (siehe Abbildung 4). Die Größe des Plasmids laßt sich als Summe der Restriktionsfragmentlängen mit 41 kb ermitteln.

Alle in diesem Beispiel aufgeführten Verfahren werden nach Standardmethoden durchgeführt (vgl. Maniatis, T., Fritsch E.F. & Sambrook, J. in "Molecular Cloning", Cold Spring Harbor, N.Y. (1982)).

**Beispiel 6**

Identifizierung der codierenden Region des Carbamat-Hydrolase-Gens durch Oligonukleotid-Hybridisierung

Durch Hybridisierung der gelelektrophoretisch aufgetrennten und auf Membranfilter transferierten Restriktionsfragmente des Plasmids pHP52 mit den in Beispiel 4 beschriebenen $^{32}P$-markierten Oligonukleotiden läßt sich die Lage der codierenden Region des Carbamat-Hydrolase-Gens auf der Restriktionskarte des Plasmids pHP52 eindeutig festlegen. In Abbildung 5 ist der hybridisierende Bereich vergrößert dargestellt. Alle drei Oligonukleotide hybridisieren mit dem zentralen Teil eines PstI-Restriktionsfragments der Größe 3,3 kb. In Abbildung 6 ist eine detaillierte Restriktionskarte des Fragments dargestellt, aus der die genauen Positionen der hybridisierenden Bereiche innerhalb des Fragments hervorgehen.

**Beispiel 7**

Klonierung des Carbamat-Hydrolase-Gens in E. coli und Nachweis der Genexpression unter lac-Promotorkontrolle

Zur Klonierung des Carbamat-Hydrolase-Gens in E. coli wird der Vektor pUC 19 (Yanish-Perron, C., Vicira, J. & Messing, J. (1985) Gene 33, 103ff.) verwendet. Die pUC 19 DNS wird durch Spaltung mit der Restriktionsnuklease PstI linearisiert und mit alkalischer Phosphatase behandelt. Die DNS des 3,3 kb langen

PstI-Restriktionsfragments des Plasmids pHP52 wird nach Spaltung der Wildtyp Plasmid-DNS mit PstI durch präparative Agarosegelelektrophorese isoliert. Die linearisierte und dephosphorylierte Vektor DNS und das 3,3 kb lange PstI-Fragment werden anschließend mit T4 DNA-Ligase ligiert. Mit dem Ligationsgemisch wird E. coli DH5$\alpha$ transformiert. Dabei werden zwei Typen von Klonen erhalten, die das Fragment in jeweils unterschiedlicher Orientierung zur Transkriptionsrichtung des lac Z'-Gens des Vektors pUC 19 enthalten. Es sind die Klone pp52 Pst und pp52 Pst inv. Die Restriktionskarte beider Klone ist in Abbildung 5 dargestellt. Die Klone des Typs E. coli pp52 Pst exprimieren nach Zugabe des Induktors Isopropyl-$\beta$-D-Thiogalaktosid zum Kulturmedium Carbamat-Hydrolase. Ohne Induktor-Zugabe (reprimierter Zustand des lac-Promotors) zu logarithmischen Kulturen der Klone pp52 Pst sowie bei reprimierten und induzierten logarithmischen Kulturen der Klone pp52 Pst inv läßt sich in Enzymeextrakten mit dem in Beispiel 3 beschriebenen Machweisverfahren keine Enzym aktivität nachweisen.

Dies bedeutet, daß das Carbamat-Hydrolase-Gen in Klonen des Typs pp52 Pst in derselben Transkriptionsrichtung (5'-3'-Orientierung) wie das lac Z' Gen des Vektors liegt. Der Arthrobacter Promotor wird offenbar in E. coli nicht (oder nur ungenügend) erkannt.

## Beispiel 8

Nukleotidsequenz des Carbamat-Hydrolase-Gens aus Arthrobacter oxidans (Stamm P52) und davon abgeleitete Proteinsequenz des Enzyms.

Die Nukleotidsequenz des Carbamat-Hydrolase-Gens wird nach der Methode von Sanger ermittelt (Sanger, F., Nicklen, S. & Coulson, A. (1977) Proc. Natl. Acad. Sci. USA, 74, 5463-5468).

Dazu werden, ausgehend von der DNS des Klons E. coli pp52 Pst, 15 Subklone in den einzelsträngigen DNS-Bakteriophagen M13 mp18 und M13 mp19 (Messing, J. (1983) Methods in Enzymol, 101, 20-78) konstruiert. Nach Transfektion in E. coli DH5$\alpha$F' wird die Sequenz der einzelsträngigen rekombinanten Desoxyribonukleinsäuren ermittelt.

In Abbildung 6 ist die Sequenzierungsstrategie des Carbamat-Hydrolase-Gens dargestellt. Insgesamt wird die Sequenz von 1864 Basenpaaren ermittelt (Strang und Gegenstrang siehe Abbildung 6).

In der Abbildung 7 wird die ermittelte Nukleotidsequenz mit der daraus abgeleiteten Proteinsequenz der Carbamat-Hydrolase gezeigt. Der Leseraster wird eindeutig durch die im Beispiel 4 beschriebenen Aminosäureteilsequenzen zweier BrCN-Spaltpeptide definiert. Der Leseraster endet mit einem TGA-Stopcodon (Nukleotid-Positionen 1789-1791 in Abbildung 7). Als Translationsstartcodon kommt ein GTG (Nukleotid-Position 340-342) in Frage. Dies ergibt einen längsten offenen Leseraster von 1479 bp (≙ 493 Aminosäuren). Alle offenen Leseraster, die mit dem üblicheren ATG Startcodon beginnen, ergeben keine Proteine passender Größe (vergleiche die Molekulargewichtsbestimmung des Proteins).

Die Festlegung des Translationsstarts auf GTG (Position 340-342) wird weiterhin gestützt durch das Vorhandensein einer deutlichen Homologieregion zur Konsensus-Sequenz für ribosomale E. coli Bindungsstellen im Abstand von 7 bp stromaufwärts des putativen GTG-Startcodons.

Alle Klonierungsschritte wurden nach Standardverfahren durchgeführt (vgl. Maniatis, T., Fritsch, E.F. & Sambrook, J. (1982) in "Molecular Cloning", Cold Spring Harbor, N.Y.). Die Sequenzreaktionen werden unter Verwendung des "Sequenase$^R$ DNA Sequencing Kits" (United States Biochemical Corporation) nach Angaben des Herstellers durchgeführt. Die Auftrennung der markierten Reaktionsprodukte erfolgte in 6% (w/v) Polyacrylamid/Harnstoff-Gelen (Maxam, A.M. & Gilbert, W. (1980) Methods Enzymol. 65, 497-559).

## Patentansprüche

1. Verfahren zur Isolierung und anschließenden Reinigung einer Carbamat-Hydrolase aus Arthrobacter oxidans, welche für die Spaltung der -OOC-N-Bindung zwischen den beiden Phenylkernen von Phenmedipham verantwortlich ist, dadurch gekennzeichnet, daß man Mikroorganismen von Arthrobacter oxidans in Nährmedien kultiviert und anschließend die Carbamat-Hydrolase durch Ultraschall-Zellaufschluß und Zentrifugation isoliert und über Anionenaustausch-Chromatographie, Gradientenelution, Ammoniumsulfatfällung, Gelfiltration und FPLC-Trennung bis zur elektrophoretischen Homogenität aufreinigt, wobei die Carbamat-Hydrolase ein pH-Optimum von pH 6,8, ein Molekulargewicht im Bereich von 50-60 kd, bestimmt über SDS-Polyacrylamid-Gelelektrophorese gemäß der Methode nach Lämmli und anschließender gängiger Gelfiltration, und einen isoelektrischen Punkt von pI = 6,2 aufweist.

2. BrCN-Spaltpeptide einer Carbamat-Hydrolase gemäß Anspruch 1, dadurch gekennzeichnet, daß sie die Aminosäuresequenzen

   Peptid I:

$H_2N$ - Ser - Asp - Glu - Phe - Ala - Asn - Leu - Asp -
- Arg - Trp - Thr - Gly - Lys - Pro - Phe - Val - Asp (Val) -
- Gly (His) - Leu - Asp - Glu - Val - Ala - Val - COOH

Peptid II:

$N_2H$ - Glu - His - Thr - Lys - Phe(Val) - Asn(Gly) - Glu - Arg(Cys) -
- Pro - Leu - Ala - Phe - Tyr - Pro -Val - Phe - Asn - Glu - COOH

aufweisen.

**3.** Verwendung der Peptide I und II gemäß Anspruch 2 zur Herstellung von synthetischen Oligonukleotiden der Sequenzen
Oligonukleotid I (Komplementärstrang):

| | | | | | |
|---|---|---|---|---|---|
| 5'- AA | A<br>GGG<br>C<br>T | C<br>TTT | A<br>GCC<br>C<br>T | A<br>GGT<br>C<br>T | CCA—3' |
| Phe | Pro | Lys | Gly | Thr | Trp |

Oligonukleotid II (Komplementärstrang):

| | | | | | | |
|---|---|---|---|---|---|---|
| 5'- CTG<br>Gln | GTC<br>Asp | CAG<br>Leu | GCC<br>Gly | GTC<br>Asp | CAC<br>Val | GAA<br>Phe |
| CGG<br>Pro | CTT<br>Lys | GCC<br>Gly | GGT<br>Thr | CCA<br>Trp | GCG<br>Arg | |
| GTC<br>Asp | - 3' | | | | | |

Oligonukleotid III (Komplementärstrang):

| | | | | | | | |
|---|---|---|---|---|---|---|---|
| 5'- TTC<br>Glu | GTT<br>Asn | GAA<br>Phe | GAC<br>Val | CGG<br>Pro | GTA<br>Tyr | GAA<br>Phe | CGC<br>Ala |

**4.** Verwendung der synthetischen Oligonukleotide gemäß Anspruch 3 zur Isolierung des Carbamat-Hydrolase-Gens.

**5.** Arthrobacter oxidans p52 (pHP52) (DSM 4044) der das 41 kb lange Plasmid pHP52 mit dem 3,3 kb langen Pst-Restriktionsfragment enthält, auf dem das Carbamat-Hydrolase-Gen mit der Sequenz gemäß Abb. 7 lokalisiert ist, der eine Carbamat-Hydrolase erzeugt, die sich mit dem Verfahren gemäß Anspruch 1 isolieren und reinigen läßt.

**6.** Arthrobacter oxidans gemäß Anspruch 5, dadurch gekennzeichnet, daß das Carbamat-Hydrolase-Gen aus einem Leseraster mit einer Nukleotidsequenz von 1479 Basenpaaren besteht.

**7.** Arthrobacter oxidans P 16/4/B (DSM 4038).

**8.** Arthrobacter oxidans P 67 (DSM 4039).

**9.** Arthrobacter oxidans P 75 (DSM 4040).

**10.** Arthrobacter oxidans P 11/1/-b (DSM 4041).

**11.** Arthrobacter oxidans P 15/4/A (DSM 4045).

**12.** Arthrobacter oxidans P 21/2 (DSM 4046).

## Claims

**1.** Process for the isolation and subsequent purification of a carbamate hydrolase from Arthrobacter oxidans which is responsible for the cleavage of the -00C-N-bond between the two benzene rings of phenmedipham characterised in that a microorganism of Arthrobacter oxidans is cultivated in a nutrient material and the carbamate hydrolase is then isolated by ultrasound cell destruction and centrifugation, and is purified by anion exchange chromatography, gradient elution, ammonium sulphate precipitation, gel filtration and FPLC separation until electrophoretic homogeneity is achieved, whereby the carbamate hydrolase shows a pH optimum of 6.8, a molecular weight in the region of 50-60 kd, determined with SDS-polyacrylamide-gel electrophoresis by the method of Lämmli and subsequent common gel filtration, and an isoelectric point of pI = 6.2.

**2.** BrCN cleaving peptide of a carbamate hydrolase, according to claim 1, characterised by having the amino acid sequences

**Peptide I:**

$H_2N$ - Ser - Asp - Glu - Phe - Ala - Asn - Leu - Asp -
- Arg - Trp - Thr - Gly - Lys - Pro - Phe - Val - Asp (Val) -
- Gly (His) - Leu - Asp - Glu - Val - Ala - Val - COOH

**Peptide II:**

$N_2H$ - Glu - His - Thr - Lys - Phe(Val) - Asn(Gly) - Glu - Arg(Cys) -
- Pro - Leu - Ala - Phe - Tyr - Pro - Val - Phe - Asn - Glu - COOH .

**3.** Use of peptides I and II according to claim 2, for the preparation of synthetic oligonucleotides having the sequences

Oligonucleotide I (complementary strand):

| | | | | | |
|---|---|---|---|---|---|
| | A | C | A | A | |
| 5' - AA | GGG | TTT | GCC | GGT | CCA-3' |
| | C | | C | C | |
| | T | | T | T | |
| Phe | Pro | Lys | Gly | Thr | Trp |

Oligonucleotide II (complementary strand):

| | | | | | | |
|---|---|---|---|---|---|---|
| 5' - CTG Gln | GTC Asp | CAG Leu | GCC Gly | GTC Asp | CAC Val | GAA Phe |
| CGG Pro | CTT Lys | GCC Gly | GGT Thr | CCA Trp | GCG Arg | |
| GTC Asp | - 3' | | | | | |

Oligonucleotide III (complementary strand):

| | | | | | | | |
|---|---|---|---|---|---|---|---|
| 5' - TTC Glu | GTT Asn | GAA Phe | GAC Val | CGG Pro | GTA Tyr | GAA Phe | CGC Ala |

**4.** Use of the synthetic oligonucleotides according to claim 3, for the isolation of the carbamate hydrolase gene.

**5.** Arthrobacter oxidans P 52 (pHP52), (DSM 4044), containing the 41 kb long plasmid pHP52 with the 3,3 kb long pst restriction fragment, in which the carbamate hydrolase gene having the sequence according to Fig. 7 is located, which produces a carbamate hydrolase that can be isolated and purified with the process according to claim 1.

**6.** Arthrobacter oxidans according to claim 5, characterised in that the carbamate hydrolase gene consists of a reading frame with a nucleotide sequence of 1479 base pairs.

**7.** Arthrobacter oxidans P 16/4/B (DSM 4038).

**8.** Arthrobacter oxidans P 67 (DSM 4039).

**9.** Arthrobacter oxidans P 75 (DSM 4040).

**10.** Arthrobacter oxidans P 11/1/-b (DSM 4041).

**11.** Arthrobacter oxidans P 15/4/A (DSM 4045).

**12.** Arthrobacter oxidans P 21/2 (DSM 4046).

**Revendications**

**1.** Procédé pour l'isolement et pour la purification d'une carbamate-hydrolase à partir de l'Arthrobacter oxidans, qui est responsable du clivage de la liaison -OOC-N- entre les deux noyaux de phényle du phenmédiphame, caractérisé en ce qu'on cultive les microorganismes Arthrobacter oxidans dans des milieux nutritifs et ensuite, on isole la carbamate-hydrolase par lyse des cellules au moyen d'ultrasons et par centrifugation, et on purifie par chromatographie par échange d'anions, par élution à gradient, par précipitation avec du sulfate d'ammonium, par filtration sur gel et par séparation FPLC jusqu'à ce qu'on obtienne l'homogénéité électrophorétique, la carbamate-hydrolase présente alors un pH optimal de 6,8, un poids moléculaire dans le domaine des 50 à 60 kd déterminé par électrophorèse sur gel SDS-polyacrylamide selon la méthode de Lämmli suivie de filtration sur gel usuelle, et un point isoélectrique pL = 6,2.

**2.** Peptides de digestion du BrCN d'une carbamate-hydrolase selon la revendication 1, caractérisés en ce qu'ils présentent des séquences d'acides aminés

Peptide I

$H_2N$ - Ser - Asp - Glu - Phe - Ala - Asn - Leu - Asp -
- Arg - Trp - Thr - Gly - Lys - Pro - Phe - Val - Asp (Val) -
- Gly (His) - Leu - Asp - Glu - Val - Ala - Val - COOH

Peptide II

$N_2H$ - Glu - His - Thr - Lys - Phe(Val) - Asn(Gly) - Glu - Arg(Cys) -
- Pro - Leu - Ala - Phe - Tyr - Pro -Val - Phe - Asn - Glu - COOH

**3.** Utilisation des peptides I et II selon la revendication 2 pour la préparation des oligonucléotides synthétiques des séquences

oligonucléotide I (brin complémentaire)

| | | | | | |
|---|---|---|---|---|---|
| 5'- AA | A<br>GGG<br>C<br>T | C<br>TTT | A<br>GCC<br>C<br>T | A<br>GGT<br>C<br>T | CCA–3' |
| Phe | Pro | Lys | Gly | Thr | Trp |

oligonucléotide II (brin complémentaire)

| | | | | | | |
|---|---|---|---|---|---|---|
| 5'- CTG | GTC | CAG | GCC | GTC | CAC | GAA |
| Gln | Asp | Leu | Gly | Asp | Val | Phe |
| CGG | CTT | GCC | GGT | CCA | GCG | |
| Pro | Lys | Gly | Thr | Trp | Arg | |
| GTC | - 3' | | | | | |
| Asp | | | | | | |

oligonucléotide III (brin complémentaire)

| | | | | | | | |
|---|---|---|---|---|---|---|---|
| 5'- TTC | GTT | GAA | GAC | CGG | GTA | GAA | CGC |
| Glu | Asn | Phe | Val | Pro | Tyr | Phe | Ala |

**4.** Utilisation d'oligonucléotides synthétiques selon la revendication 3 pour l'isolement du gène de la carbamate hydrolase.

**5.** Arthrobacter oxidans p52 (pHP52) (DSM 4044) qui contient le plasmide pHP52 de 41 kb avec le fragment de restriction Pst d'une longueur de 3,3 kb, sur lequel est localisé le gène de la carbamate-hydrolase comportant la séquence selon la figure 7, qui produit une carbamate-hydrolase que l'on peut isoler et purifier selon le procédé selon la revendication 1.

**6.** Arthrobacter oxidans selon la revendication 5, caractérisé en ce que le gène de la carbamate hydrolase comporte un cadre de lecture avec une séquence de nucléotides de 1479 paires de bases.

**7.** Arthrobacter oxidans P 16/4/B (DSM 4038)

**8.** Arthrobacter oxidans P 67 (DSM 4039)

**9.** Arthrobacter oxidans P 75 (DSM 4040)

**10.** Arthrobacter oxidans P 11/1/-b (DSM 4041)

**11.** Arthrobacter oxidans P 15/4/A (DSM 4045)

**12.** Arthrobacter oxidans P 21/2 (DSM 4046)

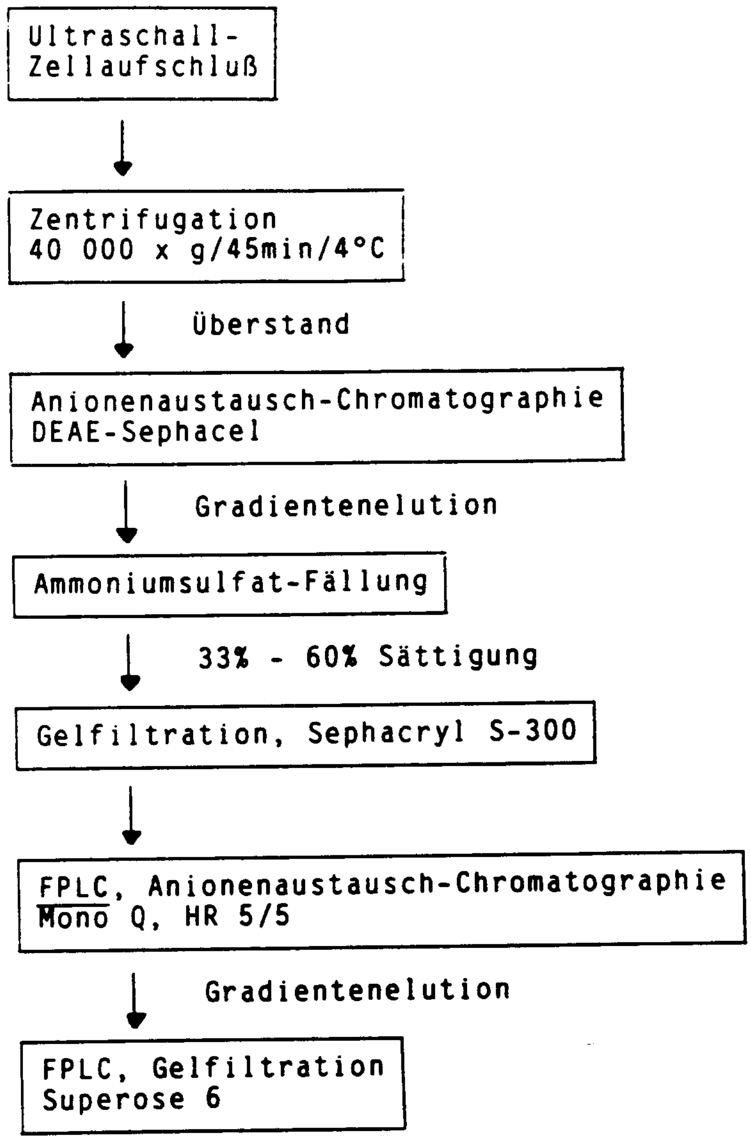
Ultraschall-
Zellaufschluß
Zentrifugation
40 000 x g/45min/4°C
Überstand
Anionenaustausch-Chromatographie
DEAE-Sephacel
Gradientenelution
Ammoniumsulfat-Fällung
33% - 60% Sättigung
Gelfiltration, Sephacryl S-300
FPLC, Anionenaustausch-Chromatographie
Mono Q, HR 5/5
Gradientenelution
FPLC, Gelfiltration
Superose 6

Abb. 1

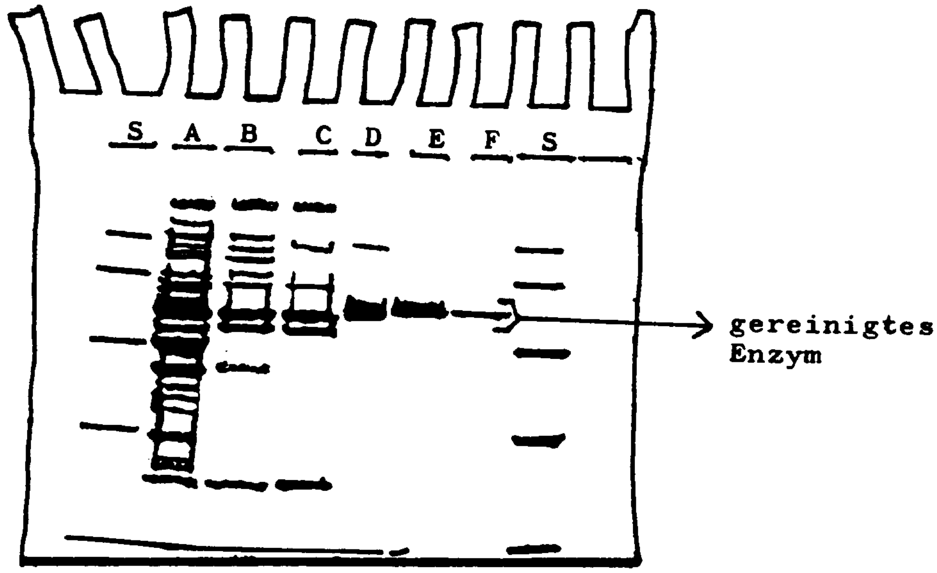


Abb. 2

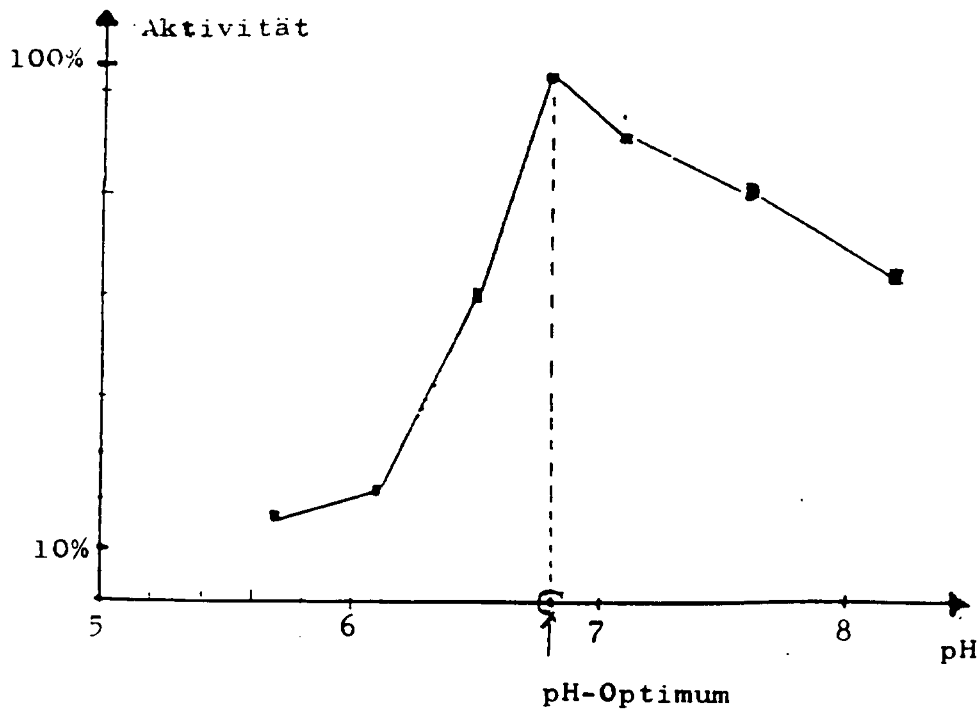


Abb. 3

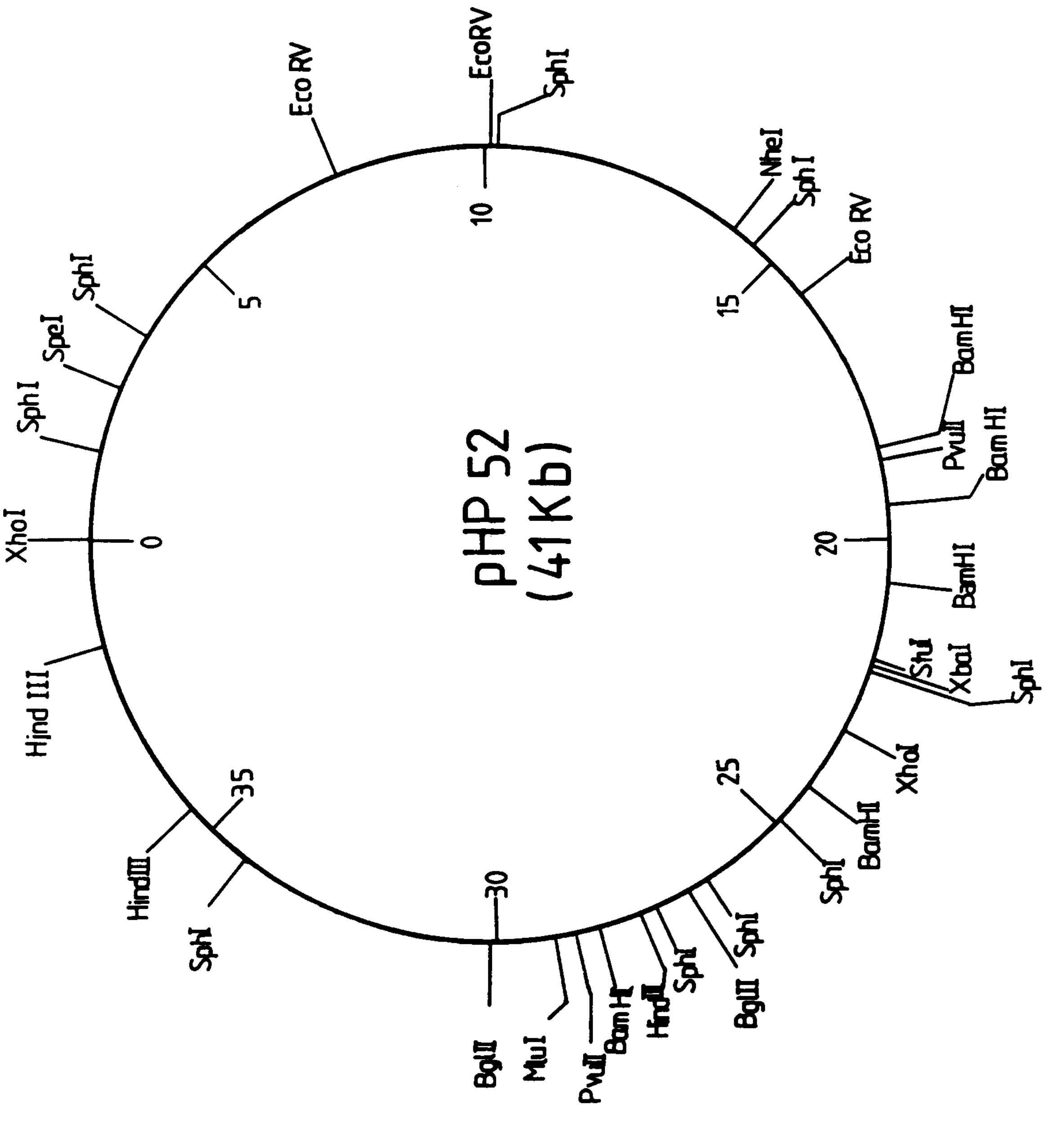
pHP 52
(41Kb)
XhoI
SphI
SpeI
SphI
EcoRV
EcoRV
SphI
NheI
SphI
EcoRV
BamHI
PvuII
BamHI
BamHI
BamHI
StuI
XbaI
SphI
XhoI
BamHI
SphI
SphI
BglII
SphI
HindIII
BamHI
PvuII
MluI
BglII
SphI
HindIII
HindIII
0
5
10
15
20
25
30
35

Abb. 4

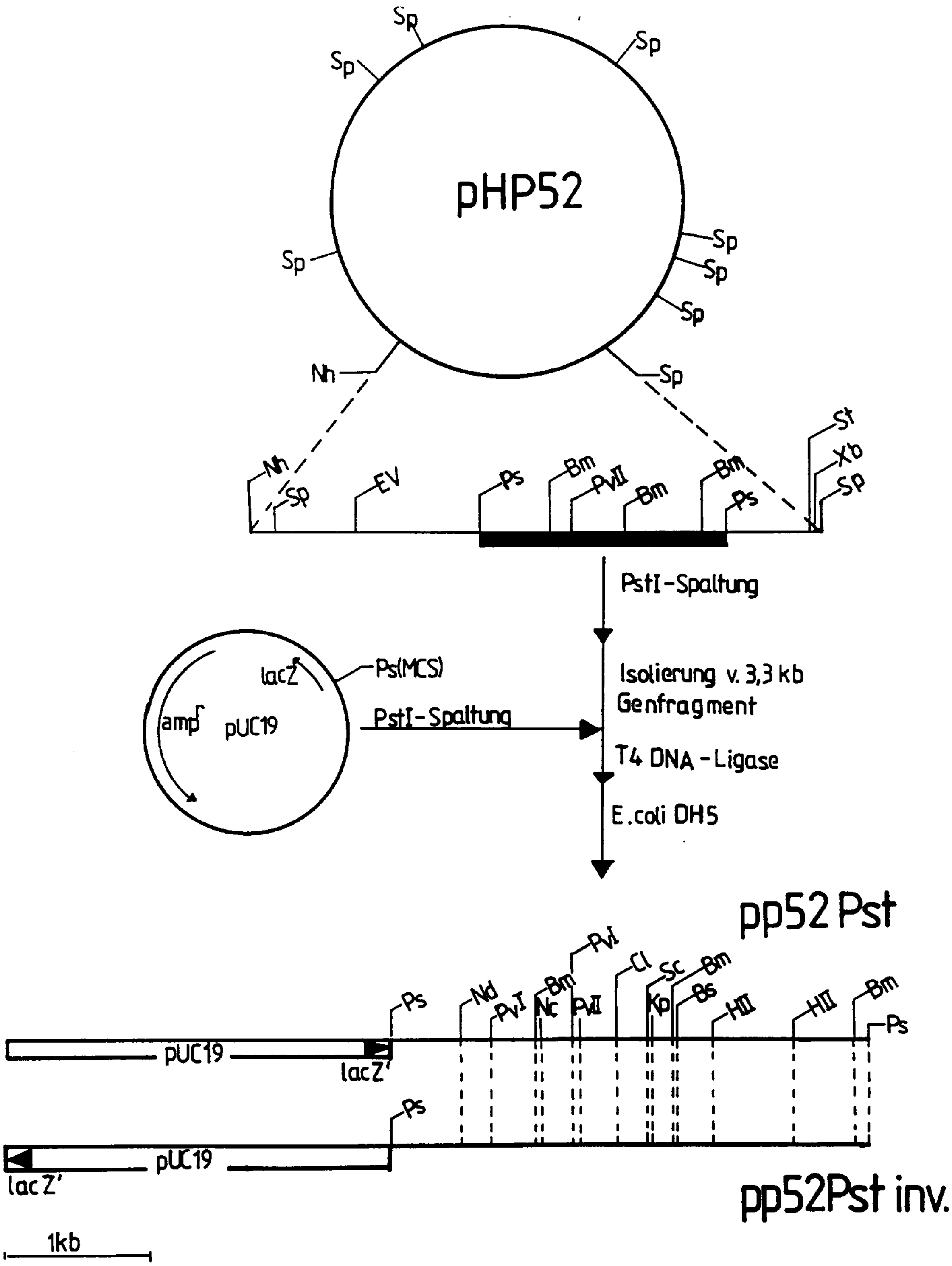
pHP52
Sp
Nh
Sp
EV
Ps
Bm
PvII
Bm
Bm
Ps
St
Xb
Sp
PstI-Spaltung
Isolierung v. 3,3 kb Genfragment
T4 DNA-Ligase
E.coli DH5
lacZ'
Ps(MCS)
amp^r
pUC19
PstI-Spaltung
pp52 Pst
Ps
Nd
PvI
Nc
Bm
PvII
PvI
Cl
Kp
Sc
Bm
Bs
HII
HII
Bm
Ps
pUC19
lacZ'
Ps
pUC19
lacZ'
pp52Pst inv.
1kb

Abb. 5

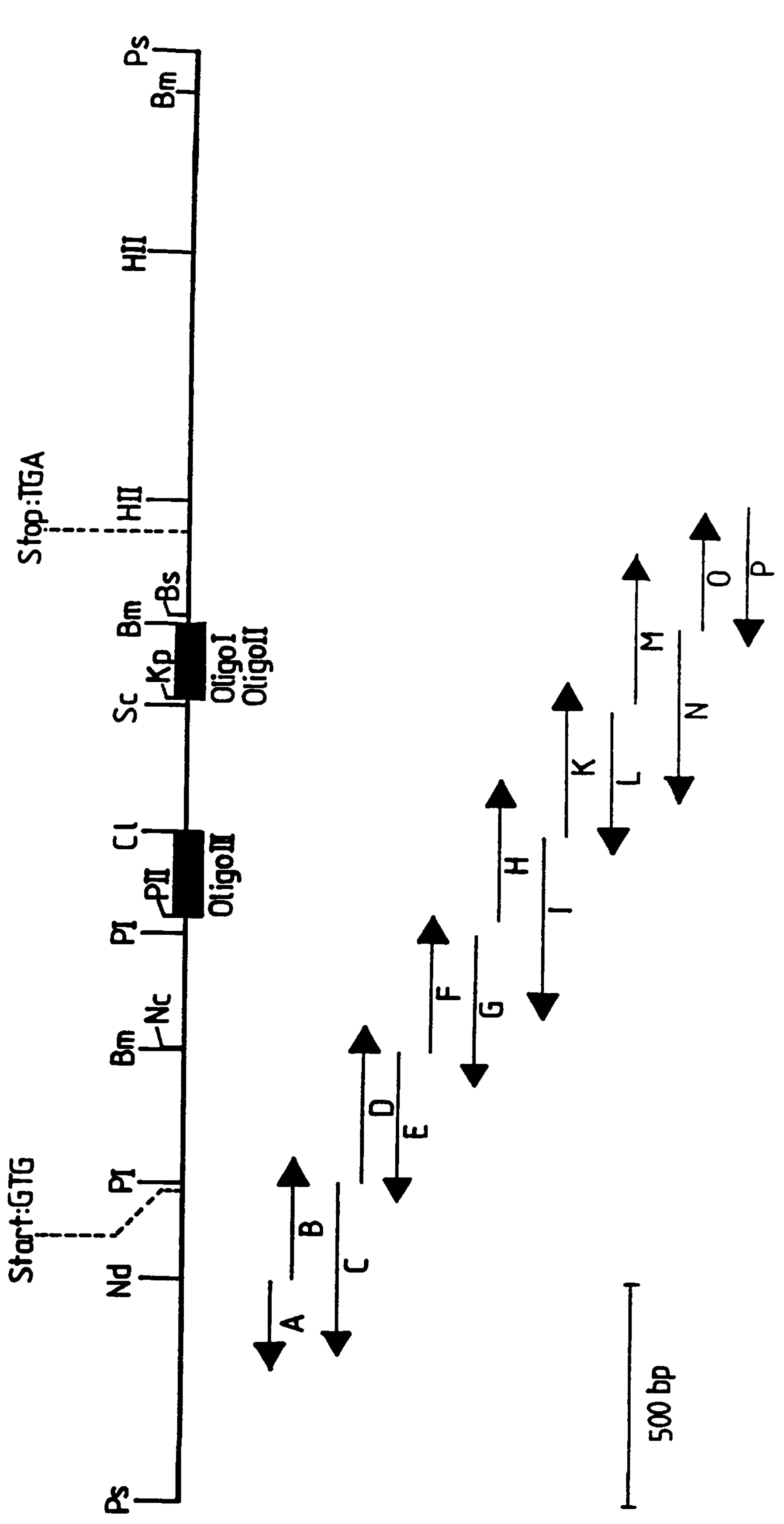
Ps
Nd
Start:GTG
PI
Bm
Nc
PI
PII
Cl
OligoIII
Sc
Kp
OligoI
OligoII
Bm
Bs
Stop:TGA
HII
HII
Bm
Ps
A
B
C
D
E
F
G
H
I
K
L
M
N
O
P
500 bp

Abb. 6

```
                              30                            60                            90                           120
TCCTTGCCAGTCACGGCACCCCAGCCAACCCGGAAGTGGCACCTGCTCGGGCACATCGGTGCGAACGCTTCGTCCTGATTCCGATGCCAACTGCTTGACGGCCGTGACACATATGTAGCA

                             150                           180                           210                           240
TAGTCGCCTAGCATGGACCCGCAGCACACCTGCTGTCGGCTCCCGCGCTATCCCCGACCAGCGCCGGTCACGGGTAGTCCTCGTGAGAGGCACCAGAACGACAACGGCGCACTGTCCCGC

                             270                           300                           330                           360
AACACGGCCGTATAACCCCACCGGGGTCCGCGCCCGAGCTAGTTCTGGCTCAACCATAAGGAGAACCTCGTGATTACCAGACCGATCGCCCACACCACCGCTGGGGACCTCGGCGGTTGC
                                                         S/D          MetIleThrArgProIleAlaHisThrThrAlaGlyAspLeuGlyGlyCys

                             390                           420                           450                           480
CTTGAAGACGGCCTGTACGTGTTCCGAGGAGTGCCGTACGCCGAGCCGCCGGTCGGCGACCTGCGGTGGCGGGCGGCGCGCCCGCACGCCGGCTGGACCGGCGTCCGCGACGCCTCCGCG
LeuGluAspGlyLeuTyrValPheArgGlyValProTyrAlaGluProProValGlyAspLeuArgTrpArgAlaAlaArgProHisAlaGlyTrpThrGlyValArgAspAlaSerAla

                             510                           540                           570                           600
TATGGTCCCTCGGCGCCGCAACCCGTGGAGCCTGGCGGCTCGCCGATCCTTGGGACACACGGCGACCCTCCGTTTGACGAGGACTGCCTGACTCTCAATCTTTGGACCCCGAACCTCGAC
TyrGlyProSerAlaProGlnProValGluProGlyGlySerProIleLeuGlyThrHisGlyAspProProPheAspGluAspCysLeuThrLeuAsnLeuTrpThrProAsnLeuAsp

                             630                           660                           690                           720
GGCGGTAGCCGGCCGGTCCTCGTCTGGATCCATGGTGGGGGCCTACTAACCGGCTCGGGAAATCTACCTAACTACGCGACCGATACCTTCGCCCGCGACGGCGACTTGGTAGGTATCTCA
GlyGlySerArgProValLeuValTrpIleHisGlyGlyGlyLeuLeuThrGlySerGlyAsnLeuProAsnTyrAlaThrAspThrPheAlaArgAspGlyAspLeuValGlyIleSer

                             750                           780                           810                           840
ATCAATTACCGGCTCGGGCCTCTTGGATTCCTCGCAGGAATGGGCGACGAGAATGTCTGGCTCACCGATCAGGTAGAGGCACTGCGCTGGATTGCAGATAACGTTGCTGCCTTCGGTGGA
IleAsnTyrArgLeuGlyProLeuGlyPheLeuAlaGlyMetGlyAspGluAsnValTrpLeuThrAspGlnValGluAlaLeuArgTrpIleAlaAspAsnValAlaAlaPheGlyGly

                             870                           900                           930                           960
GACCCGAACCGGATCACTCTCGTCGGTCAATCAGGCGGGGCATACTCGATCGCAGCGCTCGCCCAACACCCGGTCGCCCGTCAGCTGTTCCACCGCGCGATCCTACAAAGCCCACCATTC
AspProAsnArgIleThrLeuValGlyGlnSerGlyGlyAlaTyrSerIleAlaAlaLeuAlaGlnHisProValAlaArgGlnLeuPheHisArgAlaIleLeuGlnSerProProPhe

                             990                          1020                          1050                          1080
GGGATGCAACCCCATACAGTTGAAGAATCGACGGCAAGGACGAAGGCCCTGGCCCGGCATCTCGGGCACGATGACATCGAGGCCCTGCGCCATGAGCCGTGGGAGAGGCTGATTCAAGGC
GlyMetGlnProHisThrValGluGluSerThrAlaArgThrLysAlaLeuAlaArgHisLeuGlyHisAspAspIleGluAlaLeuArgHisGluProTrpGluArgLeuIleGlnGly

                            1110                          1140                          1170                          1200
ACGATAGGCGTCCTGATGGAACACACCAAATTTGGCGAATGGCCCCTGGCATTCTATCCGGTGTTCGATGAGGCAACGATACCTCGCCATCCGATTGAGTCCATTATCGATTCCGACATC
ThrIleGlyValLeuMetGluHisThrLysPheGlyGluTrpProLeuAlaPheTyrProValPheAspGluAlaThrIleProArgHisProIleGluSerIleIleAspSerAspIle
                                        Oligo III

                            1230                          1260                          1290                          1320
GAAATCATCATCGGCTGGACACGCGACGAGGGCACTTTTCCGTTTGCCTTCGACCCTCAGGTTTCACAGGCGGATCGCGATCAGGTCGAGTCATGGTTGCAGAAGCGTTTCGGAGACCAC
GluIleIleIleGlyTrpThrArgAspGluGlyThrPheProPheAlaPheAspProGlnValSerGlnAlaAspArgAspGlnValGluSerTrpLeuGlnLysArgPheGlyAspHis

                            1350                          1380                          1410                          1440
GCCGCCTCGGCCTACGAGGCTCACGCCGGCGACGGAACCAGTCCTTGGACCGTTATCGCCAACGTTGTGGGCGACGAGCTCTTTCACAGCGCTGGGTACCGGGTCGCGGACGAACGGGCA
AlaAlaSerAlaTyrGluAlaHisAlaGlyAspGlyThrSerProTrpThrValIleAlaAsnValValGlyAspGluLeuPheHisSerAlaGlyTyrArgValAlaAspGluArgAla

                            1470                          1500                          1530                          1560
ACGCGCAGACCGGTACGGGCCTATCAGTTCGACGTAGTCTCGCCCTTGTCGGACGGAGCCCTCGGCGCGGTCCACTGCATCGAAATGCCGTTCACATTTGCCAATCTCGACCGTTGGACG
ThrArgArgProValArgAlaTyrGlnPheAspValValSerProLeuSerAspGlyAlaLeuGlyAlaValHisCysIleGluMetProPheThrPheAlaAsnLeuAspArgTrpThr
                                                                                                                 Oligo I&II
                            1590                          1620                          1650                          1680
GGGAAGCCGTTCGTGGACGGCCTGGATCCAGACGTGGTGGCTCGGGTGACCAACGTGTTGCATCAGGCCTGGATCGCATTCGTCCGAACGGGAGACCCCACGCACGACCAGTTGCCGGTG
GlyLysProPheValAspGlyLeuAspProAspValValAlaArgValThrAsnValLeuHisGlnAlaTrpIleAlaPheValArgThrGlyAspProThrHisAspGlnLeuProVal

                            1710                          1740                          1770                          1800
TGGCCAACGTTCCGAGCGGACGACCCAGCGGTGTTGGTCGTCGGCGACGAGGGAGCAGAGGTGGCGCGGGATCTAGCGCGCCCGGACCACGTCAGCGTTCGGACCCTATGAGGGTCGCGG
TrpProThrPheArgAlaAspAspProAlaValLeuValValGlyAspGluGlyAlaGluValAlaArgAspLeuAlaArgProAspHisValSerValArgThrLeu+++

                            1830                          1860
GTCGCCGGGGTCTTGAGGCCGGAGGGCCTCGCGTATGCAGTGATTCGTGGATCACCGGCCAGTT
```

Abb. 7